(19) Europäisches Patentamt
European Patent Office
Office européen des brevets

(11) **EP 4 552 506 A1**

(12) **EUROPEAN PATENT APPLICATION**
published in accordance with Art. 153(4) EPC

(43) Date of publication:
**14.05.2025 Bulletin 2025/20**

(21) Application number: 23835618.2

(22) Date of filing: **07.07.2023**

(51) International Patent Classification (IPC):
*A23J 3/34* (2006.01)   *A23J 3/14* (2006.01)
*A23L 5/00* (2016.01)   *A23L 33/185* (2016.01)
*C07K 1/113* (2006.01)   *C12N 9/14* (2006.01)

(52) Cooperative Patent Classification (CPC):
**A23J 3/14; A23J 3/34; A23L 5/00; A23L 33/185;
C07K 1/113; C12N 9/14**

(86) International application number:
**PCT/JP2023/025302**

(87) International publication number:
**WO 2024/010087 (11.01.2024 Gazette 2024/02)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **07.07.2022 JP 2022110002**

(71) Applicant: **Amano Enzyme Inc.**
**Nagoya-shi**
**Aichi 460-8630 (JP)**

(72) Inventor: **SAKAI, Kiyota**
**Kakamigahara-shi, Gifu 509-0109 (JP)**

(74) Representative: **Müller-Boré & Partner**
**Patentanwälte PartG mbB**
**Friedenheimer Brücke 21**
**80639 München (DE)**

(54) **AROMA CHANGE METHOD AND TASTE ENHANCEMENT METHOD FOR VEGETABLE PROTEIN-CONTAINING COMPOSITION**

(57)   The purpose of the present invention is to provide a processing technology that changes aroma of a plant protein-containing composition and/or enhances taste thereof. The aroma of the plant protein-containing composition can be changed and/or the taste thereof can be enhanced by treating the plant protein-containing composition with a protein deamidase.

EP 4 552 506 A1

**Description**

Technical Field

The present invention relates to a technology of processing a plant protein-containing composition to change aroma thereof and/or enhance taste thereof.

Background Art

Beverages rich in nutrients such as proteins have been widely familiar to people since nutrients can be easily taken. On the other hand, against a background of the recent protein crisis, health awareness, environmental problems, and increasing awareness of animal welfare and the like, a plant protein food or drink product using a plant-derived material is becoming popular as an alternative to a food or drink product derived from an animal.

Regarding the plant protein food or drink product, various modification treatments have been studied in order to improve the existing characteristics thereof with the aim of spreading and expanding the plant protein food or drink product.

Such modification treatments have been extensively studied particularly for a soybean food or drink product. For example, PTL 1 describes that a protease-treated product obtained by subjecting a soybean protein to a protease treatment is deactivated, and a decomposition product obtained without solid-liquid separation of the deactivated product can improve the flavor of a soybean food product. PTL 2 describes that by adding a composition obtained by treating a fat component in soy milk with a lipase to a food product containing soybean as a raw material, the flavor, rich taste, and heavy taste peculiar to soybean can be imparted or enhanced.

PTL 3 describes that by performing an aminocarbonyl reaction with a protein by heating using D-psicose, a large amount of pyrazines or furanones known to impart a preferable flavor to a food product can be produced, and that this technique can also be applied to plants.

Citation List

Patent Literatures

> PTL 1: Japanese Patent Laid-open Publication No. 2007-312626
> PTL 2: Japanese Patent Laid-open Publication No. 2015-216846
> PTL 3: Japanese Patent Laid-open Publication No. 2008-048685

Summary of Invention

Technical Problem

In order to make the plant protein food or drink product widely and deeply permeate the general market, it is necessary to consider the variety of preferences of consumers, the applicability to various prepared products, or the like. In consideration of these points, there is a demand for a processing technology capable of changing characteristics such as enhancing or reducing the quality and/or level of aroma of a plant protein-containing composition or enhancing taste of a plant protein-containing composition.

Therefore, an object of the present invention is to provide a processing technology that changes aroma of a plant protein-containing composition and/or enhances taste thereof.

Solution to Problem

The present inventor has found that by treating a plant protein-containing composition with a protein deamidase, aroma of the plant protein-containing composition can be changed and/or taste thereof can be enhanced. The present inventors have conducted further studies based on the findings, leading to the completion of the present invention.

That is, the present invention provides inventions of the following aspects.

Item 1. A method for changing aroma of a plant protein-containing composition, the method including a step of treating a plant protein-containing composition with a protein deamidase.

Item 2. The method described in item 1, in which the plant protein is a protein of a plant selected from the group consisting of cereals, pulses, and nuts and seeds.

Item 3. The method described in item 1 or 2, in which the plant protein is a protein of a plant selected from the group consisting of barley, wheat, rye, sorghum, corn, soybean, lentil, fava bean, pea, chickpea, mung bean, hemp, almond, cashew nut, hazelnut, pistachio, walnut, peanut, and coconut.

Item 4. The method described in any one of items 1 to 3, in which a time for the treating is 5 hours or longer.

Item 5. The method described in any one of items 1 to 4, in which the protein deamidase is protein glutaminase.

Item 6. A method for enhancing taste of a plant protein-containing composition, the method including a step of treating a plant protein-containing composition with a protein deamidase.

Item 7. The method described in item 6, in which the plant protein is a protein of a plant selected from the group consisting of cereals, pulses, and nuts and seeds.

Item 8. The method described in item 6 or 7, in which the plant protein is a protein of a plant selected from the group consisting of barley, rice, wheat, rye, sorghum, corn, soybean, lentil, fava bean, pea, mung bean, hemp, almond, cashew nut, hazelnut, pistachio, walnut, and peanut.

Item 9. The method described in any one of items 6 to 8, in which a time for the treating is 5 hours or longer.

Item 10. The method described in any one of items 6 to 9, in which the protein deamidase is protein glutaminase.

Item 11. An aroma changing agent for a plant protein-containing composition, the agent containing a protein deamidase.

Item 12. A taste enhancing agent for a plant protein-containing composition, the agent containing a protein deamidase.

Item 13. A method for producing a processed plant protein-containing composition, the method including a step of treating a plant protein-containing composition with a protein deamidase and causing a reaction of changing aroma of the plant protein-containing composition and/or enhancing taste thereof to proceed.

Item 14. A plant protein-containing food or drink product, containing a processed plant protein-containing composition obtained by the production method described in item 13.

Advantageous Effects of Invention

According to the present invention, there is provided a processing technology that changes aroma of a plant protein-containing composition and/or enhances taste thereof.

Description of Embodiments

1. Aroma Change Method and Taste Enhancement Method for Plant Protein-Containing Composition

Both of an aroma change method and a taste enhancement method for a plant protein-containing composition of the present invention include a step of treating a plant protein-containing composition with a protein deamidase. Hereinafter, the aroma change method and the taste enhancement method for a plant protein-containing composition of the present invention will be specifically described.

1-1. Aroma Change

In the present invention, the aroma change of the plant protein-containing composition refers to a change in one or both of the quality and level of the aroma as compared with the case of not treating a plant protein-containing composition with a protein deamidase. When the quality of the aroma changes to a different aroma, this change is expressed as "imparting" of the different aroma. When the level of the same aroma changes to a different level and this different level is a higher level, this change is expressed as "enhancement" of the aroma, and when the different level is a lower level, this change is expressed as "reduction" of the aroma.

Examples of the aroma change include imparting a savory odor, reducing green aroma, imparting or enhancing green aroma, reducing the aroma of a material (plant food product material), enhancing the aroma of a material, and any combination thereof.

The "savory odor" means that the aroma of quality generated at the time of carbonization is imparted. The savory odor is given such quality of aroma, so that a pleasant aroma is generated as a whole, and is generally preferred by a wide range of preference groups.

The green aroma is a generic term for a group of volatile compounds having 6 carbon atoms such as hexanal, and is known as green leaf aroma. When the green aroma is imparted or enhanced, a characteristic green leaf aroma is produced, and thus the aroma thus changed is preferred by some preference groups as a kind of fresh aroma. On the other hand, when such a green aroma is reduced, other types of aroma of different quality stand out, or the feature of the aroma becomes a diluted plain state, and thus the aroma thus changed is preferred by a preference group or the like that repels the green aroma as a so-called raw smell of grass.

1-2. Taste Enhancement

In the present invention, the taste enhancement of the plant protein-containing composition refers to increasing the level of the taste as compared with the case of not treating a plant protein-containing composition with a protein deamidase. The taste from a qualitative viewpoint is typified by so-called five tastes (sweetness, salty taste, sourness, bitterness, and umami). Specific aspects of the enhancement of the taste include enhancing one or more of the five tastes so as to stand out, and enhancing the taste itself of the original material as a whole. Examples of the case of enhancing one or more of the five tastes so as to stand out include enhancement of sweetness, enhancement of savory taste (composite taste of bitterness and umami, or composite sense of the composite taste and savory aroma), and enhancement of sourness.

1-3. Plant Protein-Containing Composition

The plant protein-containing composition used in the present invention typically contains a plant protein material together with water, and the aspect thereof is not particularly limited. Specific examples of the aspect of the plant protein-containing composition include an aspect having fluidity, such as a liquid, a slurry, or a paste, (hereinafter, these plant protein-containing compositions of the aspect having fluidity are also collectively referred to as "liquid and the like"), and a textured or untextured solid.

Specific examples of the plant protein-containing composition include (i) a liquid and the like obtained by dispersing dry powder (an aspect of a plant protein material) of a plant (typically, a food product material) as an origin of a plant protein in water; (ii) a liquid and the like obtained by crushing and dispersing a plant as an origin of a plant protein (typically, a food product material, an aspect of a plant protein material) in water, and removing an insoluble matter derived from a skin or the like of a plant by any means such as centrifugation, filtration, filtration bag, or sieve, as necessary; (iii) a liquid and the like obtained by, for example, removing components other than the plant protein from the liquid and the like of the above (i) or (ii) to increase the concentration of the protein; (iv) a liquid and the like obtained by mixing dry powder (an aspect of a plant protein material) prepared from the liquid and the like of any one of the above (i) to (iii) with water; and (v) a textured plant protein material swollen with water.

Among these plant protein-containing compositions, those having fluidity are preferable, and those in a liquid state are more preferable. Preferred examples of the liquid plant protein-containing composition include plant milk.

The plant protein is not particularly limited, and examples thereof include proteins of plants (plant food product materials) such as cereals such as barley, rice (regardless of the degree of milling, brown rice, polished rice, and rice having the degree of milling between the brown rice and the polished rice are mentioned), wheat, rye, oats, buckwheat, sorghum, Japanese barnyard millet, foxtail millet, teff, quinoa, and corn; pulses such as soybean, lentil, fava bean, pea, chickpea, mung bean, lupine bean, and kidney bean; and nuts and seeds such as hemp, canary seed, linseed, almond, cashew nut, hazelnut, pecan nut, macadamia nut, pistachio, walnut, brazil nut, peanut, coconut, pilinut, chestnut, sesame, and pine nut. The term "hemp" in the present invention refers to a so-called industrial hemp, and specifically refers to a cultivar which does not contain tetrahydrocannabinol (THC) causing perceptual alterations or has a low THC concentration. Since industrial hemp does not contain THC or has a low concentration thereof, it has no efficacy as a perceptual alteration drug or cannot lead to abuse. These plant proteins may be used singly or in combination of a plurality of kinds thereof.

Among the origin plants of the plant protein, from the viewpoint of further enhancing the aroma changing effect, barley, wheat, rye, sorghum, corn, soybean, lentil, fava bean, pea, chickpea, mung bean, hemp, almond, cashew nut, hazelnut, pistachio, walnut, peanut, and coconut are preferable.

Among the origin plants of the plant protein, from the viewpoint of generating an aroma change that imparts a savory odor, rye, almond, coconut, hazelnut, and cashew nut are more preferable, and of these, from the viewpoint of generating an aroma change that further enhances a savory odor by reducing green aroma in addition to the above-described aroma change, hazelnut and cashew nut are further preferable.

Among the origin plants of the plant protein, from the viewpoint of generating an aroma change that imparts or enhances the green aroma, barley, soybean, lentil, mung bean, and hemp are more preferable.

Among the origin plants of the plant protein, from the viewpoint of generating an aroma change that enhances the aroma of a material, wheat, sorghum, pea, pistachio, and walnut are more preferable.

Among the origin plants of the plant protein, from the viewpoint of generating an aroma change that reduces the aroma of a material, corn, chickpea, fava bean, and peanut are more preferable, and of these, from the viewpoint of generating an aroma change that dilutes the characteristics of the aroma to make the state more plain by reducing green aroma in addition to the above-described aroma change, chickpea and fava bean are further preferable.

Among the origin plants of the plant protein, from the viewpoint of further enhancing the taste enhancing effect, barley, rice (more preferably brown rice), wheat, rye, sorghum, corn, soybean, lentil, fava bean, pea, mung bean, hemp, almond, cashew nut, hazelnut, pistachio, walnut, and peanut are preferable.

Among the origin plants of the plant protein, from the viewpoint of enhancing the sweetness, wheat, sorghum, rice (further preferably brown rice), and corn are more preferable.

Among the origin plants of the plant protein, from the viewpoint of enhancing savory taste, pistachio and hazelnut are more preferable.

Among the origin plants of the plant protein, from the viewpoint of enhancing the sourness, barley, rye, fava bean, lentil, mung bean, almond, walnut, cashew nut, peanut, and hemp are more preferable.

Among the origin plants of the plant protein, from the viewpoint of enhancing the taste of the material itself as a whole, soybean and pea are more preferable.

The content (in terms of dry weight) of the plant protein material in the plant protein-containing composition is not particularly limited, and is, for example, 0.05 to 50 wt% or 0.1 to 40 wt%, preferably 0.5 to 30 wt%, more preferably 1 to 25 wt%, further preferably 3 to 20 wt%, still more preferably 5 to 15 wt%, and further more preferably 7 to 12 wt%.

The content of the plant protein in the plant protein-containing composition is not particularly limited, and is, for example, 0.001 to 50 wt% or 0.005 to 40 wt%, preferably 0.01 to 20 wt%, more preferably 0.05 to 15 wt%, further preferably 0.1 to 10 wt%, still more preferably 0.3 to 7 wt%, and further more preferably 0.6 to 4 wt%.

The pH (25°C) of the plant protein-containing composition may be appropriately determined according to the optimal pH of the protein deamidase and the like, and is, for example, 4 to 8, preferably 5 to 7.5, and more preferably 5.8 to 7.2.

Note that, when the plant protein contained in the plant protein-containing composition is a protein of a plant having a large amount of starchy material like cereals (preferably, wheat, barley, sorghum, rye, rice, and corn), such a plant protein-containing composition is preferably treated with an amylase. The treatment with an amylase may be performed before the treatment with a protein deamidase, and may be performed simultaneously with the treatment with a protein deamidase.

Typical examples of the amylase include $\alpha$-amylase. The $\alpha$-amylase is not particularly limited, and examples thereof include $\alpha$-amylases derived from the genus Aspergillus (such as Aspergillus oryzae and Aspergillus niger), and the genus Bacillus (such as Bacillus amyloliquefaciens, Bacillus subtilis, and Bacillus licheniformis), an $\alpha$-amylase derived from the genus Bacillus is preferable, and an $\alpha$-amylase derived from Bacillus amyloliquefaciens species is more preferable.

The used amount of the $\alpha$-amylase per 1 g of the plant protein material (in terms of dry weight) is, for example, 0.1 to 100 U, preferably 0.3 to 50 U, more preferably 0.6 to 25 U, further preferably 1 to 10 U, still more preferably 1.5 to 6 U, and further more preferably 2 to 4 U.

For the activity of the $\alpha$-amylase, the amount of enzyme that reduces the color of potato starch with iodine by 10% per minute is defined as 1 unit (1 U).

1-4. Protein Deamidase

The type, origin, and the like of the protein deamidase used in the present invention are not particularly limited as long as the protein deamidase is an enzyme that exhibits an action of decomposing an amide group-containing side chain of a protein without cleaving peptide bonds and without crosslinking the protein. As long as the above action is main activity, the protein deamidase may further have an action of decomposing an amide group-containing side chain of a protein with cleaving peptide bonds and crosslinking the protein.

Examples of the protein deamidase include an enzyme that deamidates a glutamine residue in a protein and converts it into glutamic acid (for example, protein glutaminase), and an enzyme that deamidates an asparagine residue in a protein and converts it into aspartic acid (for example, protein asparaginase).

More specific examples of the protein deamidase include protein deamidases derived from the genera Chryseobacterium, Flavobacterium, Empedobacter, Sphingobacterium, Aureobacterium, Myroides, Luteimicrobium, Agromyces, Microbacterium, and Leifsonia. These protein deamidases are known, and for example, JP 2000-50887 A, JP 2001-218590 A, WO 2006/075772 A1, WO 2015/133590, or the like can be referred to. These protein deamidases may be used singly or in combination of a plurality of kinds thereof.

Among these protein deamidases, from the viewpoint of further improving the aroma changing effect and/or the taste enhancing effect, a protein deamidase derived from the genus Chryseobacterium is preferable, a protein glutaminase derived from the genus Chryseobacterium is more preferable, a protein glutaminase derived from the genus Chryseobacterium proteolyticum is further preferable, and a protein glutaminase derived from the Chryseobacterium proteolyticum 9670 strain is still more preferable.

The protein deamidase can be prepared from a culture solution of a microorganism from which the protein deamidase is derived. Specific examples of the preparation method include a method of recovering a protein deamidase from a culture solution or a bacterial cell of the above-mentioned microorganism. For example, in the case of using a microorganism that secrets a protein deamidase, an enzyme can be separated and/or purified after recovering bacterial cells from the culture solution in advance by filtration, a centrifugal treatment, or the like, as necessary. In the case of using a microorganism that does not secret a protein deamidase, an enzyme can be separated and/or purified after recovering bacterial cells from the culture solution in advance as necessary and then disrupting the bacterial cells by a pressurization treatment, an ultrasonic treatment, or the like to expose an enzyme. As an enzyme separation and/or purification method, a known protein separation and/or purification method can be used without particular limitation, and examples thereof include a centrifugal separation method, a UF concentration method, a salting-out method, and various chromatography methods using an ion-exchange resin or the like. The separated and/or purified enzyme can be powdered by a drying method such as freeze-drying or reduced-pressure drying, and can also be powdered using an appropriate excipient and/or drying aid in the drying method. The separated and/or purified enzyme can also be liquefied by adding an appropriate additive and performing filtration sterilization.

As the protein deamidase, a commercially available product can also be used, and examples of a preferred commercially available product include a protein glutaminase (derived from Chryseobacterium proteolyticum species) manufactured by Amano Enzyme Inc.

The used amount of the protein deamidase is not particularly limited, and the used amount per 1 g of the plant protein is, for example, 0.1 U or more. From the viewpoint of further improving the aroma changing effect and/or the taste enhancing effect, the used amount of the protein deamidase per 1 g of the plant protein is preferably 0.5 U or more or 1 U or more, more preferably 5 U or more, further preferably 8 U or more, and still more preferably 10 U or more. In particular, when the plant protein is derived from cereals, the used amount of the protein deamidase per 1 g of the protein derived from cereals is preferably 30 U or more, more preferably 50 U or more, further preferably 60 U or more, and still more preferably 70 U or more.

The upper limit of the used amount range of the protein deamidase per 1 g of the plant protein is not particularly limited, and is, for example, 4000 U or less, 3000 U or less, 2000 U or less, 1000 U or less, 500 U or less, 400 U or less, 300 U or less, 200 U or less, 150 U or less, 100 U or less, 90 U or less, or 80 U or less. In particular, when the plant protein is derived from nuts and seeds, the upper limit of the used amount range of the protein deamidase per 1 g of the protein derived from nuts and seeds is preferably 75 U or less, 70 U or less, 60 U or less, 55 U or less, or 50 U or less, and when the plant protein is derived from pulses, the upper limit of the used amount range of the protein deamidase per 1 g of the protein derived from pulses is preferably 40 U or less, 30 U or less, or 25 U or less.

The used amount of the protein deamidase per 1 g of the plant protein material (in terms of dry weight) is, for example, 0.01 U or more. From the viewpoint of further improving the aroma changing effect and/or the taste enhancing effect, the used amount of the protein deamidase per 1 g of the plant protein material (in terms of dry weight) is preferably 0.05 U or more or 0.1 U or more, more preferably 0.5 U or more, further preferably 0.8 U or more, still more preferably 1 U or more, and further more preferably 3 U or more. The upper limit of the used amount range of the protein deamidase per 1 g of the plant protein material (in terms of dry weight) is not particularly limited, and is, for example, 400 U or less, 300 U or less, 200 U or less, 100 U or less, 50 U or less, 40 U or less, 30 U or less, 20 U or less, 15 U or less, 10 U or less, 9 U or less, or 8 U or less.

For the activity of the protein deamidase, benzyloxycarbonyl-L-glutaminylglycine (Z-Gln-Gly) is used as a substrate, and the amount of enzyme that liberates 1 $\mu$mol of ammonia per minute is defined as 1 unit (1 U).

1-5. Treatment Conditions

The temperature at which the plant protein-containing composition is treated with a protein deamidase is not particularly limited, and can be appropriately determined by those skilled in the art according to the optimum temperature of the enzyme to be used and/or the thermal properties of the plant protein-containing composition, and the like, and is, for example, 0 to 70°C, preferably 40 to 60°C, more preferably 45 to 55°C, and further preferably 48 to 52°C.

The treatment time of the plant protein-containing composition is not particularly limited as long as the aroma of the plant protein-containing composition can be changed and/or the taste thereof can be enhanced, and is, for example, 5 hours or longer, 6 hours or longer, 7 hours or longer, 8 hours or longer, 9 hours or longer, or 10 hours or longer. From the viewpoint of further improving the aroma changing effect and/or the taste enhancing effect, the treatment time is preferably 12 hour or longer and more preferably 14 hours or longer. The upper limit of the range of the treatment time is not particularly limited, and is, for example, 24 hours or shorter, 20 hours or shorter, or 18 hours or shorter.

The plant protein-containing composition after the end of the treatment is subjected to an enzyme deactivation step and/or a filtration step as necessary, and cooled to obtain a processed plant protein-containing composition.

2. Aroma Changing Agent and Taste Enhancing Agent for Plant Protein-Containing Composition

The protein deamidase can change the aroma of the plant protein-containing composition and/or enhance the taste thereof. Therefore, the present invention also provides an aroma changing agent and a taste enhancing agent for a plant protein-containing composition, which contain a protein deamidase.

In the aroma changing agent and the taste enhancing agent of the present invention, the specific aspects of aroma change and taste enhancement, the raw material to be used, component, used amount, application target, and the like are as described in the section of "1. Aroma Change Method and Taste Enhancement Method for Plant Protein-Containing Composition".

3. Method for Producing Processed Plant Protein-Containing Composition

A method for producing a processed plant protein-containing composition of the present invention includes a step of treating a plant protein-containing composition with a protein deamidase and causing a reaction of changing the aroma of the plant protein-containing composition and/or enhancing the taste thereof to proceed.

A processed plant protein-containing composition obtained by the production method of the present invention is subjected to processing in which the aroma is changed and/or the taste is enhanced as compared with the plant protein-containing composition before the treatment.

The obtained processed plant protein-containing composition can also be prepared as a solid processed plant protein material with changed aroma and/or enhanced taste through a drying step. The drying method is not particularly limited, and examples thereof include freeze drying, vacuum drying, and spray drying. The aspect of the solid processed plant protein material may be either untextured (for example, powder, fine particles, granules, and the like) or textured.

In the method for producing a processed plant protein-containing composition of the present invention, the raw material to be used, component, used amount, treatment conditions, and the like are as described in the section of "1. Aroma Change Method and Taste Enhancement Method for Plant Protein-Containing Composition".

4. Plant Protein-Containing Food or Drink Product

A plant protein-containing food or drink product of the present invention contains a processed plant protein-containing composition obtained by "3. Method for Producing Processed Plant Protein-Containing Composition". The processed plant protein-containing composition is as described in "3. Method for Producing Processed Plant Protein-Containing Composition".

The specific form of the plant protein-containing food or drink product of the present invention can be selected from any food or drink product forms.

When the plant protein-containing food or drink product is obtained using an untextured plant protein material, examples of the specific form thereof include plant alternative milk, alternative yogurt, alternative cheese, and alternative ice cream. When the plant protein-containing food or drink product is obtained using a textured plant protein material, examples of the specific form thereof include meat-like processed food products (referring to food products imitating livestock meat, chicken meat, and/or fish minced processed food products) conforming to the forms of livestock meat, chicken meat, and/or fish minced processed food products.

The plant protein-containing food or drink product can be obtained using the processed plant protein-containing composition as it is or through any preparing step. In such a preparing step, preparing method according to the form of the plant protein-containing food or drink product is selected, and specifically, seasoning, form adjustment, molding, cooking with heat, fermentation, freezing, and/or the like can be performed.

Examples

Hereinafter, the present invention will be specifically described by means of Examples; however, the present invention is not to be construed as being limited to the following Examples.

[Enzyme to Be Used]

The following enzymes were used in the following Examples.

[Table 1]

| Enzyme to be used | Protein deamidase | Protein glutaminase derived from Chryseobacterium proteolyticum | Manufactured by Amano Enzyme Inc. |
| | Amylase | α-Amylase derived from Bacillus amyloliquefaciens | Manufactured by Amano Enzyme Inc. |

· Measurement Method of Protein Deamidase Activity

To 1 mL of a 0.2 M phosphate buffer (pH 6.5) containing 30 mM Z-Gln-Gly, 0.1 mL of a sample solution containing a protein deamidase was added, the mixture was left to stand at 37°C for 10 minutes, and then 1 mL of a 0.4 M TCA solution was added to stop the reaction. As a blank, to 1 mL of a 0.2 M phosphate buffer (pH 6.5) containing 30 mM Z-Gln-Gly, 1 mL of a 0.4 M TCA solution was added, 0.1 mL of a sample solution containing a protein deamidase was further added, and the mixture was left to stand at 37°C for 10 minutes.

The amount of ammonia generated in the reaction solution was measured for the solution obtained above using Ammonia Test Wako (FUJIFILM Wako Pure Chemical Corporation). The ammonia concentration in the reaction solution was determined from a calibration curve representing the relationship between the ammonia concentration and the absorbance (630 nm) prepared using an ammonia standard solution (ammonium chloride).

The activity of the protein deamidase was calculated from the following formula with the amount of enzyme that produces 1 $\mu$mol of ammonia per minute being defined as 1 unit (1 U). In the formula, the reaction solution amount is 2.1, the enzyme solution amount is 0.1, and Df is a dilution rate of the enzyme solution. 17.03 is a molecular weight of ammonia.

[Mathematical Formula 1]

Protein deamidase activity (U/mL)

= Ammonia concentration in reaction solution (mg/L) × (1/17.03) × (Reaction

solution amount/Enzyme solution amount) × (1/10) × Df

· $\alpha$-Amylase Activity Measurement Method

After 10 mL of a 1 wt% potato starch substrate solution (0.1 mol/L of acetic acid (pH 5.0)) was warmed at 37°C for 10 minutes, 1 mL of a sample solution containing an $\alpha$-amylase was then added, and the mixture was immediately shaken. This solution was left to stand at 37°C for 10 minutes, 1 mL of this solution was then added to 10 mL of a 0.1 mol/L hydrochloric acid solution, and the mixture was immediately shaken. Next, 0.5 mL of this solution was weighed, 10 mL of 0.0002 mol/L iodine solution (the Japanese pharmacopoeia) was added, the mixture was shaken, and then an absorbance (AT) at a wavelength of 660 nm was measured using water as a control. Separately, an absorbance (AB) was measured by the same operation adding 1 mL of water instead of the sample solution. The amount of enzyme that reduces the color of potato starch with iodine by 10% per minute was defined as 1 unit (1 U).

[Mathematical Formula 2]

$\alpha$-Amylase activity (U/g, U/mL) = (AB - AT)/AB × 1/W

AT: Absorbance of reaction solution
AB: Absorbance of blank solution
W: Amount (g or mL) of sample in 1 mL of sample solution

[Plant Protein Material to Be Used]

The following plant protein materials were used in the following Examples.

[Table 2]

| Plant protein material to be used | Product name | Origin plant | Protein content (weight basis) | Manufacturer |
|---|---|---|---|---|
| | Wheat whole grain flour | Wheat | 14.0 ± 2.0% | TOMIZ/cuoca (TOMIZAWA SHOUTEN) |
| | Barley whole grain flour | Barley | 13.0 ± 1.0% | TOMIZ/cuoca (TOMIZAWA SHOUTEN) |
| | White sorghum flour | Sorghum | 9.60% | NAKANO INDUSTRY CO., LTD. |
| | Rye whole grain flour | Rye | 7.5 ± 1.0% | TOMIZ/cuoca (TOMIZAWA SHOUTEN) |
| | Brown rice flour | Brown rice | 7.10% | Kodama Ltd. |
| | Corn flour | Corn | 6.60% | Kodama Ltd. |
| | Sprouted soybean flour | Soybean | 36.70% | Kodama Ltd. |
| | Yellow Split Peas | Pea | 31.70% | Waitrose |
| | Organic chickpea | Chickpea | 20% | OHSAWA JAPAN |
| | Fava bean | Fava bean | 26% | Mameyano Soko Chikara |
| | Lentil | Lentil | 23.20% | Spice Specialty Store SPICE MARKET |
| | Mung whole green bean | Mung bean | 37.65% | ohtsuya |
| | Almond | Almond | 10.30% | TOMIZ (TOMIZAWA SHOUTEN) |
| | Pistachio | Pistachio | 17.40% | SUKOYAKA SHOUTEN |
| | Coconut | Coconut | 7.00% | Alishan Pty. Ltd. |
| | Walnut | Walnut | 14.60% | TOMIZ (TOMIZAWA SHOUTEN) |
| | Hazelnut | Hazelnut | 13.36% | TOMIZ (TOMIZAWA SHOUTEN) |
| | Cashew nut | Cashew nut | 19.80% | KOJIMAYA |
| | Peanut | Peanut | 26.50% | Kobeno Omamesan MINOYA |
| | Hemp | Hemp seed[※] | 29.50% | Imagawa Seika |

※ Hemp seed is derived from industrial hemp not containing THC.

[Test Example]

(1) Production of Processed Plant Protein-Containing Composition

A liquid plant protein-containing composition (pH at 25°C: 6 to 7) was prepared by the following procedure. A liquid plant protein-containing composition derived from wheat, barley, sorghum, rye, brown rice, corn, or soybean was prepared by weighing a powder plant protein material shown in Table 2, adding water to have a powder content of 10% (w/w), and performing a mixer treatment. Other liquid plant protein-containing compositions were prepared by weighing a powder product obtained by crushing a plant protein material shown in Table 2 with a mill, adding water to have a powder content of 10% (w/w), and performing a mixer treatment.

A protein deamidase (protein glutaminase) was added to the liquid plant protein-containing composition in an amount shown in Table 3 and Table 4, and an enzyme treatment was performed at 50°C for 16 hours while shaking. The reaction solution amount was 10 mL. Note that, in the case of using a plant protein material derived from cereals (wheat, barley, sorghum, rye, brown rice, or corn), α-amylase (3 U per 1 g of the plant protein material) was also added, and the enzyme treatment was performed at 50°C for 16 hours while shaking together with the protein deamidase (protein glutaminase). After the enzyme treatment, an enzyme deactivation treatment was performed at 100°C for 10 minutes and cooling to room temperature was performed. Thereby, a processed plant protein-containing composition of Examples was obtained.

As a comparative target, a processed plant protein-containing composition for comparison was obtained by performing the same operation except that a protein deamidase (protein glutaminase) was not added.

(2) Evaluation of Aroma Changing Effect and Evaluation of Taste Enhancing Effect

The aroma property and taste property of the processed plant protein-containing composition of Examples and the processed plant protein-containing composition for comparison were checked by three trained panelists. How the aroma of the processed plant protein-containing composition of Examples was changed in terms of quality and level as compared with the comparative one was evaluated. The results are shown in Table 3. As for the enhancement of the taste of the processed plant protein-containing composition of Examples, what kind of taste was enhanced as compared with the comparative one was evaluated. The results are shown in Table 4.

[Table 3]

| | Plant protein-containing composition | | | Used amount of protein deamidase | | Aroma changing effect | |
|---|---|---|---|---|---|---|---|
| | Origin plant | Material(※) content (W/W) | Plant protein content (W/W) | Per 1 g of material(※) | Per 1 g of plant protein | Quality | Level |
| Example 1 | Wheat | 10% | 1.4% | 5 U | 36.4~35.0 U | Not changed | Enhanced |
| Example 2 | Barley | 10% | 1.3% | 5 U | 38.8~38.1 U | Green aroma | Enhanced |
| Example 3 | Sorghum | 10% | 1.0% | 5 U | 52.1 U | Not changed | Enhanced |
| Example 4 | Rye | 10% | 0.8% | 5 U | 67.3~66.0 U | Savory | Enhanced(*) |
| Example 5 | Corn | 10% | 0.7% | 5 U | 75.8 U | Not changed | Reduced |
| Example 6 | Soybean | 10% | 3.7% | 5 U | 13.6 U | Green aroma | Enhanced |
| Example 7 | Pea | 10% | 3.2% | 5 U | 15.8 U | Not changed | Enhanced |
| Example 8 | Chickpea | 10% | 2.0% | 5 U | 25 U | Not changed | Reduced(‡) |
| Example 9 | Fava bean | 10% | 2.6% | 5 U | 19.2 U | Not changed | Reduced(‡) |
| Example 10 | Lentil | 10% | 2.3% | 5 U | 21.6 U | Green aroma | Enhanced |
| Example 11 | Mung bean | 10% | 3.8% | 5 U | 13.3 U | Green aroma | Enhanced |
| Example 12 | Almond | 10% | 1.0% | 5 U | 48.5 U | Savory | Enhanced(*) |
| Example 13 | Pistachio | 10% | 1.7% | 5 U | 28.7 U | Not changed | Enhanced |
| Example 14 | Coconut | 10% | 0.7% | 5 U | 71.4 U | Savory | Enhanced(*) |
| Example 15 | Walnut | 10% | 1.5% | 5 U | 34.2 U | Not changed | Enhanced |
| Example 16 | Hazelnut | 10% | 1.3% | 5 U | 37.4 U | Green aroma | Reduced(†) |
| Example 17 | Cashew nut | 10% | 2.0% | 5 U | 25.3 U | Green aroma | Reduced(†) |
| Example 18 | Peanut | 10% | 2.7% | 5 U | 18.9 U | Not changed | Reduced |
| Example 19 | Hemp | 10% | 3.0% | 5 U | 16.9 U | Green aroma | Enhanced |

(※) Refer to the plant protein material of Table 2.
(*) The effect of changing the aroma was particularly high in that a savory odor accepted by a wide range of preference groups was enhanced.
(†) The effect of changing the aroma was particularly high in that the savory odor of the material was remarkable as a result of reducing green aroma.
(‡) The effect of changing the aroma was particularly high in that the strong green aroma of the material was also reduced with a reduction in the overall aroma.

As shown in Examples 1 to 19, various kinds of aroma could be changed by treating the indicated plant protein-containing composition with a protein deamidase. In particular, as shown in Examples 4, 12, and 14, in the case of using the plant protein-containing compositions derived from rye, almond, and coconut, the effect of changing the aroma was particularly high in that a savory odor accepted by a wide range of preference groups was enhanced. As shown in Examples 16 and 17, in the case of using the plant protein-containing compositions derived from hazelnut and cashew nut, the effect of changing the aroma was particularly high in that the savory odor of the material was remarkable as a result of reducing green aroma. As shown in Examples 8 and 9, in the case of using the plant protein-containing compositions derived from chickpea and fava bean, the effect of changing the aroma was particularly high in that the strong green aroma of the material was also reduced with a reduction in the overall aroma.

[Table 4]

| | Plant protein-containing composition | | | Used amount of protein deamidase | | Taste enhancing effect |
|---|---|---|---|---|---|---|
| | Origin plant | Material(※) content (W/W) | Plant protein content (W/W) | Per 1 g of material(※) | Per 1 g of plant protein | Enhanced taste |
| Example 20 | Wheat | 10% | 1.4% | 5 U | 36.4~35.0 U | Sweetness[*] |
| Example 21 | Barley | 10% | 1.3% | 5 U | 38.8~38.1 U | Sourness |
| Example 22 | Sorghum | 10% | 1.0% | 5 U | 52.1 U | Sweetness[*] |
| Example 23 | Rye | 10% | 0.8% | 5 U | 67.3~66.0 U | Sourness |
| Example 24 | Brown rice | 10% | 0.7% | 5 U | 70.4 U | Sweetness[*] |
| Example 25 | Corn | 10% | 0.7% | 5 U | 75.8 U | Sweetness[*] |
| Example 26 | Soybean | 10% | 3.7% | 5 U | 13.6 U | Taste of raw material |
| Example 27 | Pea | 10% | 3.2% | 5 U | 15.8 U | Taste of raw material |
| Example 28 | Fava bean | 10% | 2.6% | 5 U | 19.2 U | Sourness |
| Example 29 | Lentil | 10% | 2.3% | 5 U | 21.6 U | Sourness |
| Example 30 | Mung bean | 10% | 3.8% | 5 U | 13.3 U | Sourness |
| Example 31 | Almond | 10% | 1.0% | 5 U | 48.5 U | Sourness |
| Example 32 | Pistachio | 10% | 1.7% | 5 U | 28.7 U | Savory taste[*] |
| Example 33 | Walnut | 10% | 1.5% | 5 U | 34.2 U | Sourness |
| Example 34 | Hazelnut | 10% | 1.3% | 5 U | 37.4 U | Savory taste[*] |
| Example 35 | Cashew nut | 10% | 2.0% | 5 U | 25.3 U | Sourness |
| Example 36 | Peanut | 10% | 2.7% | 5 U | 18.9 U | Sourness |
| Example 37 | Hemp | 10% | 3.0% | 5 U | 16.9 U | Sourness |

(※) Refer to the plant protein material of Table 2.
(*) The effect of enhancing the taste was particularly high in that the taste accepted by a wide range of preference groups was enhanced.

As shown in Examples 20 to 37, various kinds of taste could be enhanced by treating the indicated plant protein-containing composition with a protein deamidase. In particular, as shown in Examples 20, 22, 24, 25, 32, and 34, in the case of using the plant protein-containing compositions derived from wheat, sorghum, brown rice, corn, pistachio, and hazelnut, the effect of enhancing the taste was particularly high in that sweetness and savory taste, which are the tastes accepted by a wide range of preference groups, were enhanced.

**Claims**

1.    A method for changing aroma of a plant protein-containing composition, the method comprising a step of treating a plant protein-containing composition with a protein deamidase.

2.    The method according to claim 1, wherein the plant protein is a protein of a plant selected from the group consisting of cereals, pulses, and nuts and seeds.

3.    The method according to claim 1, wherein the plant protein is a protein of a plant selected from the group consisting of barley, wheat, rye, sorghum, corn, soybean, lentil, fava bean, pea, chickpea, mung bean, hemp, almond, cashew nut, hazelnut, pistachio, walnut, peanut, and coconut.

4. The method according to claim 1, wherein a time for the treating is 5 hours or longer.

5. The method according to claim 1, wherein the protein deamidase is protein glutaminase.

6. A method for enhancing taste of a plant protein-containing composition, the method including a step of treating a plant protein-containing composition with a protein deamidase.

7. The method according to claim 6, wherein the plant protein is a protein of a plant selected from the group consisting of cereals, pulses, and nuts and seeds.

8. The method according to claim 6, wherein the plant protein is a protein of a plant selected from the group consisting of barley, rice, wheat, rye, sorghum, corn, soybean, lentil, fava bean, pea, mung bean, hemp, almond, cashew nut, hazelnut, pistachio, walnut, and peanut.

9. The method according to claim 6, wherein a time for the treating is 5 hours or longer.

10. The method according to claim 6, wherein the protein deamidase is protein glutaminase.

11. An aroma changing agent for a plant protein-containing composition, the agent comprising a protein deamidase.

12. A taste enhancing agent for a plant protein-containing composition, the agent comprising a protein deamidase.

13. A method for producing a processed plant protein-containing composition, the method comprising a step of treating a plant protein-containing composition with a protein deamidase and causing a reaction of changing aroma of the plant protein-containing composition and/or enhancing taste thereof to proceed.

14. A plant protein-containing food or drink product, comprising a processed plant protein-containing composition obtained by the production method according to claim 13.

## INTERNATIONAL SEARCH REPORT

| International application No. |
| --- |
| **PCT/JP2023/025302** |

### A.　CLASSIFICATION OF SUBJECT MATTER

*A23J 3/34*(2006.01)i; *A23J 3/14*(2006.01)i; *A23L 5/00*(2016.01)i; *A23L 33/185*(2016.01)i; *C07K 1/113*(2006.01)i; *C12N 9/14*(2006.01)i

FI:　A23J3/34; A23J3/14; A23L5/00 J; A23L5/00 M; A23L33/185; C07K1/113; C12N9/14

According to International Patent Classification (IPC) or to both national classification and IPC

### B.　FIELDS SEARCHED

Minimum documentation searched (classification system followed by classification symbols)

A23J3/34; A23J3/14; A23L5/00; A23L33/185; C07K1/113; C12N9/14

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C.　DOCUMENTS CONSIDERED TO BE RELEVANT

| Category* | Citation of document, with indication, where appropriate, of the relevant passages | Relevant to claim No. |
| --- | --- | --- |
| X | WO 2022/014542 A1 (AMANO ENZYME INC.) 20 January 2022 (2022-01-20) claims, paragraphs [0008]-[0061] | 1-14 |
| X | WO 2022/059755 A1 (AMANO ENZYME INC.) 24 March 2022 (2022-03-24) claims, paragraphs [0012]-[0043] | 1-14 |
| X | WO 2022/092242 A1 (AMANO ENZYME INC.) 05 May 2022 (2022-05-05) claims, paragraphs [0012]-[0064] | 1-5, 11, 13-14 |
| X | WO 2022/102723 A1 (AMANO ENZYME INC.) 19 May 2022 (2022-05-19) paragraphs [0096]-[0105] | 14 |
| X | WO 2022/059754 A1 (AMANO ENZYME INC.) 24 March 2022 (2022-03-24) paragraphs [0042]-[0048] | 14 |
| P, X | CN 115413711 A (GRASSGEN ROBOTS GROUP PTY LTD) 02 December 2022 (2022-12-02) claims, paragraphs [0012], [0021]-[0091] | 6-7, 10, 13-14 |

☐ Further documents are listed in the continuation of Box C.　　☑ See patent family annex.

| * | Special categories of cited documents: |
| --- | --- |

"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **05 September 2023** | **12 September 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/025302**

| Patent document cited in search report | | | Publication date (day/month/year) | Patent family member(s) | | | Publication date (day/month/year) |
|---|---|---|---|---|---|---|---|
| WO | 2022/014542 | A1 | 20 January 2022 | EP 4180528 A1 claims, paragraphs [0007]-[0063] CN 115715156 A | | | |
| WO | 2022/059755 | A1 | 24 March 2022 | (Family: none) | | | |
| WO | 2022/092242 | A1 | 05 May 2022 | CN 114521591 A claims, paragraphs [0012]-[0064] | | | |
| WO | 2022/102723 | A1 | 19 May 2022 | (Family: none) | | | |
| WO | 2022/059754 | A1 | 24 March 2022 | (Family: none) | | | |
| CN | 115413711 | A | 02 December 2022 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007312626 A
- JP 2015216846 A
- JP 2008048685 A
- JP 2000050887 A
- JP 2001218590 A
- WO 2006075772 A1
- WO 2015133590 A